# EUROPEAN PATENT APPLICATION

(11) **EP 4 052 738 A1**
(43) Date of publication of application: **07.09.2022**
(21) Application number: 21160864.1
(22) Date of filing: 05.03.2021
(51) Int. Cl.: A61M 1/16, A61M 1/28

(54) **PERITONEAL DIALYSIS FLUID FROM DRINKING WATER**

(71) Applicant: Bellco S.r.l., 41037 Mirandola (IT)
(72) Inventor: VERATTI, Andrea, 41122 Modena (IT); ROMA, Arianna, 41037 Mirandola (IT); RICCI, Maria Chiara, 41037 Modena (IT); PUVIANI, Fabrizio, 41037 San Felice Sul Panaro (IT)
(74) Representative: Maschio, Antonio

(57) **Abstract**

The disclosure relates to systems and methods for generating peritoneal dialysis fluid from a potable water source, such as drinking water. The systems and methods use mainly disposable components that can be replaced for each treatment. The systems and methods purify water from the water source, generate concentrates from dry materials using the purified water, and cycle the generated peritoneal dialysis fluid into and out of the patient.

## Description

### FIELD

The disclosure relates to systems and methods for generating peritoneal dialysis fluid from a potable water source, such as drinking water. The systems and methods use disposable components that can be replaced for each treatment. The systems and methods purify water from the water source, generate concentrates from dry materials using the purified water, and cycle the generated peritoneal dialysis fluid into and out of the patient.

### BACKGROUND

Peritoneal Dialysis is a type of renal therapy whereby a catheter is placed in the peritoneal cavity and peritoneal dialysis fluid is introduced directly into the peritoneal cavity. Blood is cleaned inside the patient using the patient's own peritoneum as a type of dialysis membrane. However, because fluid is directly introduced into a human body, the fluid used for peritoneal dialysate is generally required to be free of biological and chemical contaminants. The peritoneal dialysate should also contain specified concentrations of solutes and cations for biocompatibility and for performing membrane exchange. Because each peritoneal dialysis cycle can include several exchanges of liters of fluid each exchange, storage of purified peritoneal dialysis fluid is problematic. Further, systems and methods that generate peritoneal dialysis fluid as needed from a potable water source require constant disinfecting of complicated and expensive components, reducing the ability for patients to use peritoneal dialysis.

As such, there is a need for systems and methods for generating peritoneal dialysis fluid from a potable water source that can be operated by a patient at home. The need extends to systems and methods that use largely disposable components, reducing complexity of the system and operation.

### SUMMARY OF THE INVENTION

The problem to be solved by the present invention is online preparation of peritoneal dialysis fluid using disposable components. The solution is to use disposable and relatively inexpensive components in a disposable cassette that can be changed before each use.

The first aspect of the invention relates to a system. In any embodiment, the system can include a water purification module fluidly connectable to a water source; and a disposable cassette; the disposable cassette having a first connector fluidly connectable to the water purification module; the disposable cassette further including a) a first fluid line fluidly connected to the first connector; b) at least one concentrate line fluidly connectable to a first concentrate source and fluidly connected to the first fluid line; c) at least one osmotic agent line fluidly connectable to a first osmotic agent source and fluidly connected to the first fluid line; and d) at least a second connector fluidly connectable to a patient.

In any embodiment, the disposable cassette can include at least one microbial filter.

In any embodiment, the disposable cassette can include at least one pump.

In any embodiment, the disposable cassette can include a mixing bag fluidly connected to the first fluid line.

In any embodiment, the disposable cassette can include a purified water bag fluidly connected to the first fluid line upstream of the at least one concentrate line and at least one osmotic agent line.

In any embodiment, the system can include a heater for heating the purified water bag.

In any embodiment, the at least one osmotic agent line can include a first osmotic agent line fluidly connectable to a first osmotic agent source and a second osmotic agent line fluidly connectable to a second osmotic agent source.

In any embodiment, the at least one concentrate line can include a first concentrate line fluidly connectable to a first concentrate source and a second concentrate line fluidly connectable to a second concentrate source.

In any embodiment, the water source can be a drinkable water source.

In any embodiment, the system can include a receiving compartment for receiving the disposable cassette.

The features disclosed as being part of the first aspect of the invention can be in the first aspect of the invention, either alone or in combination, or follow any arrangement or permutation of any one or more of the described elements. Similarly, any features disclosed as being part of the first aspect of the invention can be in a second or third aspect of the invention described below, either alone or in combination, or follow any arrangement or permutation of any one or more of the described elements.

The second aspect of the invention relates to a method of using the system of the first aspect of the invention. In any embodiment, the method can include the steps of: flowing water from the water source through the water purification module and into the first fluid line; flowing a concentrate from the at least one concentrate source into the first fluid line; flowing an osmotic agent concentrate from the at least one osmotic agent source into the first fluid line; and flowing fluid from the first fluid line into a mixing bag.

In any embodiment, the method can include the step of determining a mass of fluid in the mixing bag; and wherein the step of flowing the concentrate into the first fluid line comprises flowing the concentrate until a mass of fluid in the mixing bag increases by a preset amount; and wherein the step of flowing the osmotic agent concentrate into the first fluid line comprises flowing the osmotic agent concentrate until a mass of fluid in the mixing bag increases by a preset amount.

In any embodiment, the method can include the step of heating the mixing bag after flowing the fluid from the first fluid line into the mixing bag.

In any embodiment, the method can include the step of flowing water into the at least one concentrate source to generate the concentrate and flowing water into the at least one osmotic agent source to generate the osmotic agent concentrate.

In any embodiment, the method can include the step of flowing water from the water purification module to a purified water bag prior to flowing the water into the at least one concentrate source and the at least one osmotic agent source.

In any embodiment, the method can include the step of determining a mass of water in the purified water bag; wherein the step of flowing water into the at least one concentrate source includes flowing a predetermined mass of water into the at least one concentrate source; and wherein the step of flowing water into the at least one osmotic agent source includes flowing a predetermined mass of water into the at least one osmotic agent source.

The features disclosed as being part of the second aspect of the invention can be in the second aspect of the invention, either alone or in combination, or follow any arrangement or permutation of any one or more of the described elements. Similarly, any features disclosed as being part of the second aspect of the invention can be in the first or third aspect of the invention, either alone or in combination, or follow any arrangement or permutation of any one or more of the described elements.

The third aspect of the invention is drawn to a method of using the system of the first aspect of the invention. In any embodiment, the method can include the steps of: flowing water from the water source through the water purification module; flowing the water from the water purification module into a purified water bag fluidly connected to the first fluid line; flowing water from the purified water bag into the at least one concentrate source to generate a concentrate; and flowing water from the purified water bag into the at least one concentrate source to generate an osmotic agent concentrate.

In any embodiment, at least one concentrate source can initially contain solid material, and the at least one osmotic agent source can initially contain solid material.

In any embodiment, the method can include the step of determining a mass of water in the purified water bag prior to flowing the water into the at least one concentrate source and the at least one osmotic agent source.

In any embodiment, the step of flowing water from the purified water bag to the at least one concentrate source can include flowing the water until the mass of water in the purified water bag decreases by a preset amount.

In any embodiment, the step of flowing water from the purified water bag to the at least one osmotic agent source can include flowing the water until the mass of water in the purified water bag decreases by a preset amount.

The features disclosed as being part of the third aspect of the invention can be in the third aspect of the invention, either alone or in combination, or follow any arrangement or permutation of any one or more of the described elements. Similarly, any features disclosed as being part of the third aspect of the invention can be in the first or second aspect of the invention described below, either alone or in combination, or follow any arrangement or permutation of any one or more of the described elements.

### BRIEF DESCRIPTION OF THE DRAWINGS

FIG. 1 is a diagram showing portions of a system for generating peritoneal dialysis fluid.
FIG. 2 is a flow diagram of a peritoneal dialysis fluid generation system.
FIG. 3 is a non-limiting embodiment of a water purification module.
FIG. 4 is a flow chart showing steps of generating concentrates for use with a peritoneal dialysis fluid generation system.
FIG. 5 is a flow chart showing steps of using concentrates to generate peritoneal dialysis fluid.
FIG.'s 6A-E illustrate the steps in using a peritoneal dialysis fluid generation system.
FIG. 7 is a non-limiting example of a peritoneal dialysis fluid generation system.

### DETAILED DESCRIPTION

Unless defined otherwise, all technical and scientific terms used have the same meaning as commonly understood by one of ordinary skill in the art.

The articles "a" and "an" are used to refer to one to over one (i.e., to at least one) of the grammatical object of the article. For example, "an element" means one element or over one element.

The term "comprising" includes, but is not limited to, whatever follows the word "comprising." Use of the term indicates the listed elements are required or mandatory but that other elements are optional and may be present.

A "concentrate" is a solution of one or more solutes in water. The concentrate solution can have a solute concentration greater than that to be used in dialysis.

A "concentrate source" is a source of one or more solutes. The concentrate source can have one or more solutes that has a solute concentration greater than the solute concentration to be used for dialysis.

A "concentrate line" is a fluid line fluidly connecting a concentrate source with a second component or fluid line.

A "connector" is a component that forms a fluid connection between two or more components.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "determining," "determines," and the like, generally refer to, in the broadest reasonable interpretation, any process or method for obtaining or coming to a decision, value, number, or finding, for any one or more value, output, parameter, or variable, by any means applicable to the relevant parameter being determined.

A "disposable cassette" is a set of components attached together that are intended to be used and then disposed of.

A "drinkable water source" is a water source from which potable water can be obtained.

The terms "flowing" or to "flow" refer to moving a fluid through a flow path with by any means.

A "fluid" is a liquid substance optionally having a combination of gas and liquid phases in the fluid. Notably, a liquid, as used herein, can therefore also have a mixture of gas and liquid phases of matter.

A "fluid line" can refer to a tubing or conduit through which a fluid or fluid containing gas can pass. The fluid line can also contain air during different modes of operation such as cleaning or purging of a line.

The term "fluidly connectable" refers to the ability of providing for the passage of fluid, gas, or combination thereof, from one point to another point. The ability of providing such passage can be any connection, fastening, or forming between two points to permit the flow of fluid, gas, or combinations thereof. The two points can be within or between any one or more of compartments of any type, modules, systems, components, and rechargers.

The term "fluidly connected" refers to a particular state such that the passage of fluid, gas, or combination thereof, is provided from one point to another point. The connection state can also include an unconnected state, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The terms "generate," "generating," "is generated," and the like refer to forming a solution or substance from constituent parts.

A "heater" is a component capable of raising the temperature of a substance, container, or fluid.

The terms "heating" or to "heat" refer to raising the temperature of a substance, fluid, gas, or container.

The term "initially" refers to a state of a component or system prior to a process.

The term "mass" refers to a measure of an amount of matter of a substance.

The term "microbial filter" refers to a device inhibiting passage of microbes or fragments of microbes such as endotoxins in a fluid or solution while allowing the passage of the fluid or solution.

A "mixing bag" is a container into which separate components of a fluid can be added for mixing to form a final fluid.

An "osmotic agent" is a substance dissolved in water capable of driving a net movement of water by osmosis across a semi-permeable membrane due to concentration differences of the osmotic agent on each side of the semi-permeable membrane. Osmotic agents can include glucose, icodextrin, dextrose, and any other suitable substance or compound known to those of skill in the art for use as an osmotic agent in peritoneal dialysis.

An "osmotic agent line" is a fluid line fluidly connecting an osmotic agent source with a second component or fluid line.

An "osmotic agent source" refers to a source of osmotic agents in solid and/or solution form.

A "patient" or "subject" can be a member of any animal species, preferably a mammalian species, optionally a human. The subject can be an apparently healthy individual, an individual suffering from a disease, or an individual being treated for a disease. In certain embodiments, the patient can be a human, sheep, goat, dog, cat, mouse or any other animal.

"Peritoneal dialysis" is a therapy wherein a dialysate is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution has captured sufficient amounts of the waste components the fluid is removed. This cycle can be repeated for several cycles each day or as needed.

The term "preset amount" refers to a value or range for a variable that is set prior to a process causing a change in the variable.

The term "processor" or "microprocessor" as used is a broad term and is to be given an ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. In any embodiment of the first, second, third, and fourth invention, the terms can include ROM ("read-only memory") and/or RAM ("random-access memory") associated therewith.

The term "programmed," when referring to a processor, can mean a series of instructions that cause a processor to perform certain steps.

The term "pump" refers to any device that causes the movement of fluids or gases by applying suction or pressure.

A "purified water bag" is a container into which already purified water can be added for later use.

A "receiving compartment" can be a compartment, section, or chamber within a larger component into which a smaller component or module can be positioned.

The terms "solid" or "solid material" refer to a material in the solid phase of matter, and can include crystalline, powdered, or any other form of solid material.

The term "upstream" refers to a position of a first component in a flow path relative to a second component, wherein fluid, gas, or a combination thereof, will pass by the first component prior to the second component during normal operation. The first component can be said to be "upstream" of the second component, while the second component is "downstream" of the first component.

The term "water purification module" refers to a component or collection of components capable of removing biological or chemical contaminants from water.

The term "water source" refers to any source from which potable or non-potable water can be obtained.

### Peritoneal Dialysis System

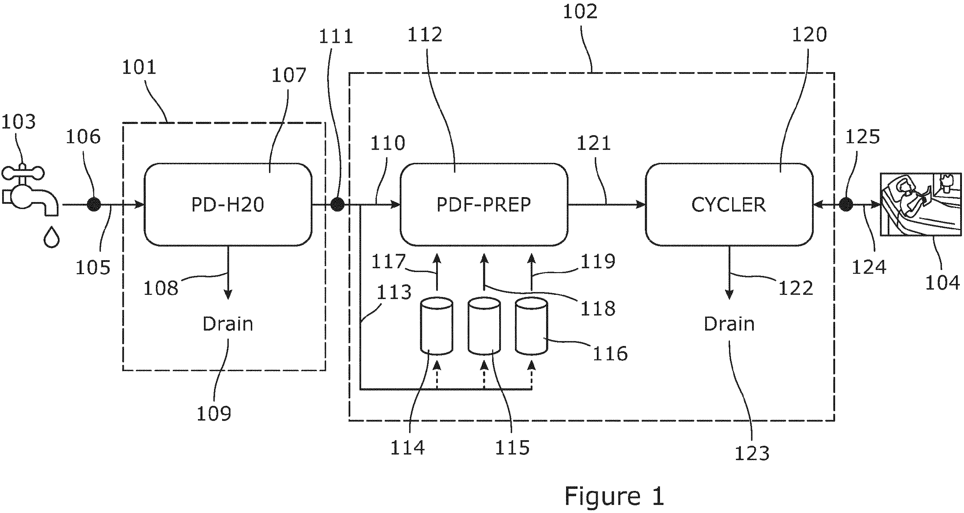

FIG. 1 illustrates a system for generating and using peritoneal dialysis fluid from water and powder or concentrates. The system can include a water purification portion **101** and a peritoneal dialysis fluid preparation portion **102.** The water purification portion **101** connects to a drinkable water source **103** through fluid line **105** at connector **106.** However, in certain embodiments, the water source need not be drinkable water. The purpose of the water purification portion **101** is to produce water suitable for peritoneal dialysis fluid. The purpose of the peritoneal dialysis fluid preparation portion **102** is to generate a required amount of peritoneal dialysis fluid and to fill and drain the patient. In certain embodiments, the water purification portion **102** can include a graphical user interface. The user can input data and receive information from the water purification position **102** through the graphical user interface. The water purification portion **101** and peritoneal dialysis fluid preparation portion **102** can communicate together through any type of wired or wireless communication. After purification, the water is flowed into the peritoneal dialysis fluid preparation portion **102** through fluid line **110** and connector **111.** In certain embodiments, the peritoneal dialysis fluid preparation portion **102** can also include cycler **120** connected to a peritoneal dialysis fluid generation module **112** through connector **121.** Fluid is flowed into and out of patient **104** from cycler **120** through fluid line **124** and connector **125.**

The water purification portion **101** and peritoneal dialysis fluid preparation portion **102** can exchange water suitable for generating peritoneal dialysis fluid through a fluid line. The size of the fluid line can vary depending on the needs of the user. In certain embodiments the fluid line connecting the water purification portion **101** and peritoneal dialysis fluid preparation portion **102** can be 20 feet or more, allowing the water purification portion **101** to be stored in a separate room from the peritoneal dialysis fluid preparation portion **102.** For example, the water purification portion **101** can be stored in a bathroom, while the peritoneal dialysis fluid preparation portion **102** is stored in a patient's bedroom.

The water purification portion **101** includes a water purification module **107** for purifying water from drinkable water source **103.** The drinkable water source **103** can be a source of potable water including a purified water source. Purified water can refer to any source of water treated to remove at least some biological or chemical contaminants. The drinkable water source **103** can alternatively be a non-purified water source, such as tap water, wherein the water from the drinkable water source **103** can be purified by the system as described.

The water purification module **107** can include one or more components to purify water from the drinkable water source **103.** In certain embodiments, the water purification module **107** can be a sorbent cartridge. The sorbent cartridge can include aluminum oxide for removal of fluoride and heavy metals. The sorbent cartridge can have a first layer of aluminum oxide, a second layer of activated carbon and a third layer of an ion exchange resin. The sorbent cartridge can be sized depending on the needs of the user, with a larger sized sorbent cartridge allowing for more exchanges before the sorbent cartridge must be replaced. The sorbent cartridge can also include activated carbon. The activated carbon operates to adsorb non-ionic molecules, organic molecules, and chlorine from the water, along with some endotoxins or bacterial contaminants. In certain embodiments, the sorbent cartridge can include activated carbon, activated alumina, and potentially other components that work primarily by physical and chemical adsorption, combined with one or more ion exchange materials. The ion exchange materials can be any known material in the art, but preferably the ion exchange materials will release hydrogen and hydroxyl ions in exchange for other cations and anions in solution, resulting in water formation by the exchange process.

The water purification module **107** can additionally include a microbial filter and/or a particulate filter. A microbial filter can further reduce the amount of bacterial contaminants present in the fluid from the drinkable water source **103** or fluid line **105.** Optionally, an ultrafilter can be included to remove endotoxins from the fluid. A particulate filter can remove particulate matter from the fluid. In certain embodiments, the water purification module **107** can include an optional UV light source for further purification and sterilization of the water prior to adding osmotic agents or ion concentrates.

Alternatively, the water purification module **107** can be any component capable of removing contaminants from the water in the drinkable water source **103,** including any one or more of a sorbent cartridge, reverse osmosis module, nanofilter, combination of cation and anion exchange materials, activated carbon, activated alumina, silica, or silica-based columns. Waste fluid from the water purification module **107** can be flowed to a drain **109** through fluid line **108.**

After passing through the water purification portion **101** of the system, the purified water can be flowed into the peritoneal dialysis fluid preparation portion **102.** The peritoneal dialysis fluid preparation portion **102** includes a peritoneal dialysis fluid generation system **112.** The peritoneal dialysis fluid generation system **112** uses concentrates that are mixed together and diluted with water to generate peritoneal dialysis fluid. To generate the concentrates, water from the water purification module **107** can be flowed into one or more concentrate sources containing dry powders through fluid line **113.** The system illustrated in FIG. 1 includes a first concentrate source **114,** a second concentrate source **115,** and a third concentrate source **116.** The water is mixed with the dry powders in each concentrate source to generate concentrates. The concentrate generated by concentrate source **115** can be added to the peritoneal dialysis fluid generation system **112** through fluid line **117;** the concentrate generated by concentrate source **116** can be added to the peritoneal dialysis fluid generation system **112** through fluid line **118;** and the concentrate generated by concentrate source **117** can be added to the peritoneal dialysis fluid generation system **112** through fluid line **119.** Additional water from water purification module **107** is also flowed into the peritoneal dialysis fluid generation system **112** through fluid line **110** to generate peritoneal dialysis fluid having proper concentrations of all solutes.

Although shown with three concentrate sources in FIG. 1, the system can use any number of concentrate sources, including 2, 3, 4, 5, 6 or more concentrate sources. In certain embodiments, the system can use separate osmotic agent concentrate sources and ion concentrate sources, allowing the osmotic agent concentration in the generated peritoneal dialysis fluid to be varied independent of the ion concentrations. The system can also include multiple osmotic agent concentrate sources and multiple ion concentrate sources, as described.

In certain embodiments, the peritoneal dialysis fluid preparation portion **102** can also include a cycler **120.** The generated peritoneal dialysis fluid can be flowed from the peritoneal dialysis fluid generation system **112** through fluid line **121** to cycler **120.** The cycler **120** can infuse the peritoneal dialysis fluid into the patient **104** through fluid line **124** and connector **125.** Spent peritoneal dialysis fluid can be drained from the patient **104** through the same fluid line **124** prior to infusion. The spent peritoneal dialysis fluid can be flowed to a drain **123** through fluid line **122.** One of skill in the art will understand that drain **123** and drain **109** can be the same drain.

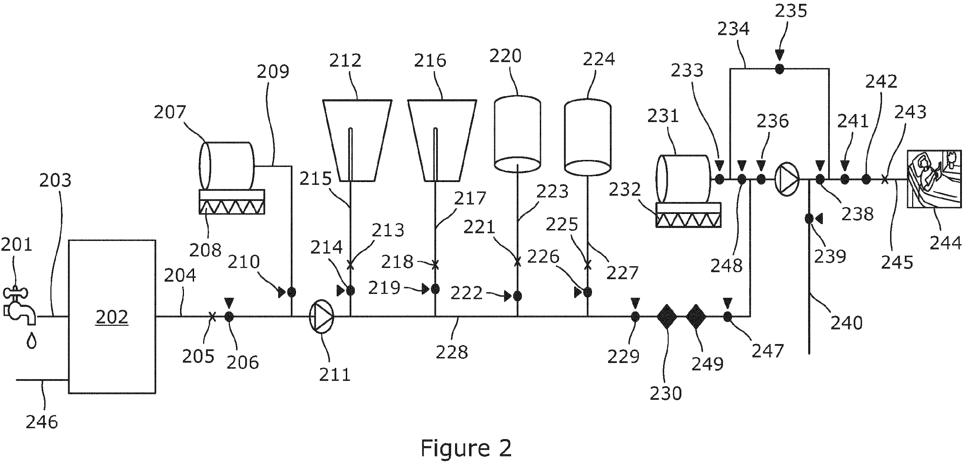

FIG. 2 illustrates the components of the peritoneal dialysis system. As described, water from a drinkable water source **201** can be flowed into a water purification module **202** through fluid line **203.** As described, water purification module **202** can purify the incoming drinking water to generate purified water for use in peritoneal dialysis. Wastewater from water purification module **202** can be directed to a drain (not shown) through drain line **246.**

Purified water from water purification module **202** can be flowed through fluid line **204** to connector **205.** Connector **205** connects fluid line **204** to fluid line **228** of a peritoneal dialysis fluid generation portion of the system. Valve **206** controls the movement of fluid from the water purification module **202** into fluid line **228.** Pump **211** can provide the force for flowing fluid throughout the system.

To generate peritoneal dialysis fluid, valve **206** and valve **210** can be opened, while the other valves of the system are closed to direct purified water through fluid line **209** into purified water bag **207.** Purified water bag **207** can be any container capable of storing the purified water prior to use and need not be a bag. For example, a rigid container can be used in place of purified water bag **207.** Purified water bag **207** can be placed on top of or hanged on load cell **208.** Load cell **208** determines the mass of water in purified water bag **207** and can be in communication with a processor of a control system programmed for controlling the peritoneal dialysis system described. In certain embodiments, load cell **208** can also include a heater. The heater can heat the purified water to a desired temperature prior to generating concentrates, as elevated temperature may improve dissolution of the solid material. The purified water bag **207** allows purified water to be generated and used as a batch. In certain embodiments, the water purification module **202** can be operated independently of the other components. For example, the water purification module **202** can be operated to fill purified water bag **207** at any time, with the peritoneal dialysis fluid generation portions connected only when needed.

In certain embodiments, the system can generate peritoneal dialysis fluid starting from dry powders. To generate concentrates from dry powders, purified water is added to containers containing the dry powders. As described, a heater may be used to heat the purified water prior to adding the purified water to the concentrate sources. Heating the purified water may accelerate dissolution of the concentrates. Further, because the solutes may be more soluble at higher temperature, heating the purified water prior to adding the purified water to the concentrate sources may allow for a higher final concentration in the reconstituted concentrate. The higher final concentration can allow a smaller concentrate source. For example, sodium bicarbonate is significantly more soluble at an elevated temperature than at room temperature. The volume needed for the same amount of sodium bicarbonate to be dissolved is therefore lower at an elevated temperature, requiring less space. To generate a concentrate, valve **210** and valve **214** can be opened to direct fluid from purified water bag **207** through connector **213** and fluid line **215** into concentrate source **212.** As described, concentrate source **212** can initially contain a dry powder. With a known mass of dry powder in concentrate source **212,** a concentrate of known concentration can be generated by controlling the amount of purified water added to concentrate source **212.** The control system can control pump **211** to flow purified water from purified water bag **207** into concentrate source **212** until the mass of water in purified water bag **207** has decreased by a preset amount. Knowing the mass of water flowed into concentrate source **212** and the mass of dry powder initially in the concentrate source **212,** the system can determine the concentration of solutes in the generated concentrate.

A second concentrate can be generated by closing valve **214** and opening valve **219** to flow purified water from purified water bag **207** through connector **218** and fluid line **217** into concentrate source **216.** As described, load cell **208** measures the mass of water flowed out of water bag **207** and into concentrate source **216** to generate a concentrate of known concentration.

To generate an osmotic agent concentrate, valve **219** can be closed and valve **222** opened to flow purified water from purified water bag **207** through connector **221** and fluid line **223** into osmotic agent source **220.** The control system can control pump **211** as described to generate an osmotic agent concentrate of known concentration in osmotic agent source **220.** Similarly, valve **222** can be closed and valve **226** opened to flow purified water from purified water bag **207** through connector **225** and fluid line **227** into osmotic agent source **224.**

FIG. 2 is illustrated with a first concentrate source **212,** a second concentrate source **216,** a first osmotic agent source **220,** and a second osmotic agent source **224.** However, more or fewer concentrate and osmotic agent sources can be used. For example, all ion concentrates can be present in a single concentrate source. Alternatively, each constituent of the peritoneal dialysis fluid can be in a separate concentrate source. In certain embodiments, a single osmotic agent source can be used. However, with two osmotic agent sources, two different osmotic agents can be used. For example, glucose or dextrose can be included for shorter dwell period cycles, while icodextrin can be included for longer dwell period cycles. Any number of concentrate or osmotic agent sources can be included in the system.

To generate peritoneal dialysis fluid, valve **214,** valve **229,** valve **247,** valve **236,** valve **238,** valve **235,** and valve **233** can be opened while pump **237** is operated to flow the concentrate from concentrate source **212** through fluid line **228** and into mixing bag **231.** Similar to purified water bag **207,** mixing bag **231** can be placed on top of load cell **232.** As described, the concentrates generated are of known concentration. The control system can operate pump **237** until a predetermined mass of fluid has been flowed into mixing bag **231.** If a second concentrate source **216** is used, valve **214** can be closed and valve **219** opened to flow a predetermined mass of concentrate from concentrate source **216** into mixing bag **231.** Similarly, by closing valve **219** and opening valve **222,** a predetermined mass of osmotic agent concentrate can be flowed from osmotic agent source **220** into mixing bag **231.** A predetermined mass of an osmotic agent concentrate from osmotic agent source **224** can be added to mixing bag **231** by opening valve **226** while the other valves are closed. In certain embodiments, only a single osmotic agent source can be used for each cycle. However, in certain embodiments, two different osmotic agents can be combined. Once each concentrate has been added to mixing bag **231,** additional water can be added to dilute the peritoneal to a desired concentration. The control system can open valve **210** to flow water from purified water bag **207** to mixing bag **231.** Valve **210,** valve **229,** valve **233,** valve **248,** and valve **247** can be opened and pump **211** can flow the purified water to mixing bag **231.** The amount of water needed to be added depends on the concentrations of each of the concentrates added to mixing bag **231,** which as described, can be controlled by the system. Microbial filter **230** and a second microbial filter **249** can be included in fluid line **228** to remove any biological contaminants from the fluid prior to addition to the mixing bag **231.** A heater (not shown) can be included in contact with the mixing bag **231** to heat the fluid prior to infusion into the patient.

To remove spent peritoneal dialysis fluid from a patient **244,** valve **241,** valve **242,** valve **235,** valve **236,** valve **248,** and valve **239** can be opened to direct fluid from a peritoneal cavity of patient **244** through fluid line **245** and fluid line **234** and into drain line **240.** Drain line **240** can be connected to a drain (not shown). Once the spent peritoneal dialysis fluid has been drained from patient **244,** new peritoneal dialysis fluid can be infused into the patient **244** from mixing bag **231.** Valve **233,** valve **236,** valve **238,** valve **242,** valve **248,** and valve **241** can be opened and pump **237** operated to direct fresh peritoneal dialysis fluid from mixing bag **231** through connector **243** and patient infusion line **245** into the peritoneal cavity of the patient **244.**

In certain embodiments, the system can include a disposable cassette having the peritoneal dialysis fluid generation portion. For example, fluid line **228** between connector **205** and connector **243** can be part of the disposable cassette. Pump **211,** pump **237** and the valves shown can all be disposable. Because the system can use load cell **208** to determine the volume of water used to generate the concentrates and load cell **232** to determine the volume of fluid added to the mixing bag **231,** accurate volumetric pumps or flow meters are not necessary. However, one of skill in the art will understand that various sensors, such as flow sensors, pressure sensors, conductivity sensors, and/or osmotic agent sensors can be included. In certain embodiments, purified water bag **207** and mixing bag **231** can be part of the disposable cassette. Although shown as part of fluid line **228,** microbial filters **230** and **249** can alternatively be outside of the disposable cassette, downstream of connector **243.**

For use, the user need only connect the disposable cassette at connectors **205** and **243.** The concentrate sources can be prefilled with dry powders or liquids. The user can connect each concentrate source to the disposable cassette. For example, concentrate source **212** can be connected through connector **213,** concentrate source **216** can be connected through connector **218,** osmotic agent source **220** can be connected at connector **221,** and osmotic agent source **224** can be connected at connector **225.** In certain embodiments, each concentrate source and osmotic agent source can also be disposable, leaving the only durable portion as water purification module **202.** Because all components other than the water purification module **202** can be disposable, heating and sterilization of the other components is not necessary.

In certain embodiments, the disposable cassette can include all components other than water purification module **202.** The water purification module **202** can include a receiving compartment into which all or part of the disposable cassette can be placed.

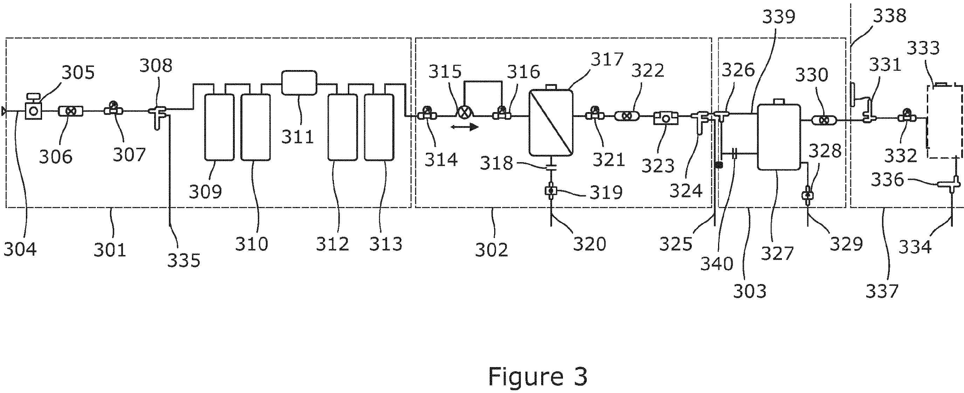

FIG. 3 shows a non-limiting embodiment of a water purification module. The water purification module can include multiple stages, including a pretreatment stage **301,** a reverse osmosis stage **302,** an electrodeionization stage **303,** and an ultrafiltration stage **337.** Water from a water source (not shown) can be flowed into the pretreatment stage **301** through fluid line **304.** The water can pass through a pretreatment pressure valve **305,** which can act as a pressure reducer, to ensure that the pressure of the incoming water is not too high. A conductivity sensor **306** and pressure sensor **307** can be included to measure the conductivity and pressure of the incoming water. The water can then reach valve **308.** If the conductivity or pressure of the water is outside of a predetermined range, the water can be flowed through fluid line **335** to a drain (not shown). If the incoming water is within a usable conductivity and pressure range, the water is flowed into a carbon/sediment filter **309.** The carbon/sediment filter **309** can remove larger particulate matter from the incoming water. The water then passes through water softener **310** and UV lamp **311** to remove some biological contaminants. The water passes through carbon block **312** and carbon block **313** to remove additional non-ionic matter from the water. Using two carbon blocks provides further purification and redundancy.

After passing through the pretreatment stage **301,** the water can enter reverse osmosis stage **302.** Pressure sensor **314** can measure the pressure of water entering reverser osmosis stage **302.** Pump **315** can provide the driving force for flowing water through the middle and later stages of the water purification module. In certain embodiments, an additional pump (not shown) can be used to flow fluid through the pretreatment stage **301.** Alternatively, water pressure from the water source can be used to flow water through the pretreatment stage **301.** A second pressure sensor **316** can determine the pressure of the water after passing through pump **315.** The water can be flowed through reverse osmosis module **317** to remove additional chemical contaminants from the water. Waste water from reverse osmosis module **317** can be flowed through reverse osmosis orifice **318** and valve **319** to the drain through fluid line **320.** Water exiting reverse osmosis module **317** can flow through pressure sensor **321,** conductivity sensor **322** and flow sensor **323** to measure the pressure, conductivity, and flow rate of the water. If the pressure, flow rate, or conductivity is outside of a predetermined range, the water can be flowed through fluid line **325** from valve **324** to a drain. If the pressure and conductivity are within a predetermined range, the water can be flowed into electrodeionization stage **303.**

Water entering electrodeionization stage **303** can pass through T-junction **326** and into electrodeionization unit **327.** The electrodeionization unit **327** removes any ionic species from the source water. The electrodeionization unit **327** can include two separate streams. A main feedwater stream, which is being purified, can enter the electrodeionization unit **327** through fluid line **339.** A concentrate and electrode stream can enter the electrodeionization unit **327** through fluid line **340.** The concentrate and electrode stream removes the electrochemical waste generated by the electrodeionization unit **327,** allowing regeneration of the resin. Waste water from the electrodeionization unit **327** can be flowed through valve **328** and fluid line **329** to a drain. The water exiting the electrodeionization unit **327** can be flowed through conductivity sensor **330** into the ultrafiltration stage **337** at valve **331.** If the conductivity of the water is within an acceptable range, the water can be flowed through pressure sensor **332** into ultrafiltration unit **333.** If the conductivity is outside of the predetermined range, the water can be flowed through fluid line **338** to a drain. Ultrafiltration unit **337** removes microbiological contaminants from the water to place the water within a range suitable for peritoneal dialysis fluid production. The water, now purified, can flow into the rest of the peritoneal dialysis system (not shown). Waste water from the ultrafiltration unit **337** can flow through valve **336** and fluid line **334** to a drain. FIG. 3 shows a single possible embodiment of a water purification module. As described, other components and arrangements can be used to purify incoming water.

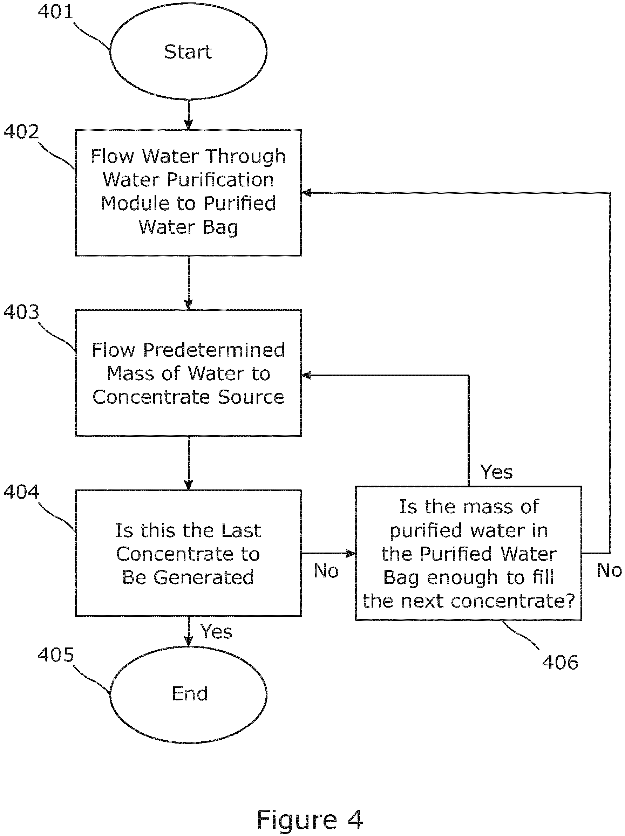

FIG. 4 is a flow chart showing the steps used to generate concentrates from drinking water. The process can start in step **401.** In step **402,** water can be flowed through a water purification module, as described, and flowed into a purified water bag. In step **403,** a predetermined mass of purified water can be flowed from the purified water bag into a concentrate source. As described, the purified water bag can be placed on a load cell to measure the mass. The mass of water added to the concentrate source is simply the decrease in mass of the purified water bag. By adding a known mass of water to a known mass of dry powder or liquid material in the concentrate source, a concentrate of known concentration is formed by step **403.** In step **404,** the system can determine whether all needed concentrates have been formed. If so, the process can end in step **405.** If not, the system can determine whether the mass of water remaining in the purified water bag is enough to fill the next concentrate. If so, the method can continue in step **403,** generating the next concentrate. If not, the method can revert to step **402,** generating additional purified water to use for the next concentrate.

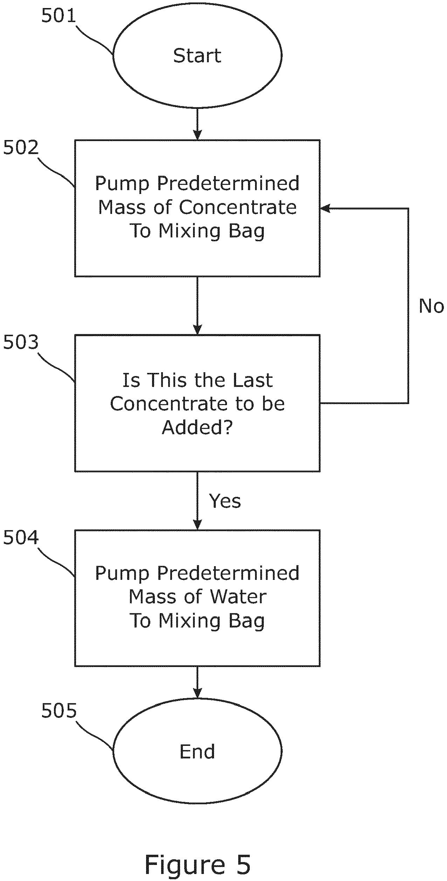

FIG. 5 is a flow chart showing the steps used to generate peritoneal dialysis fluid using the concentrates generated by the method of FIG. 4. The process can start in step **501.** In step **502,** a concentrate can be flowed from a concentrate source into a mixing bag. As described, the concentration of solutes in each concentrate is known by using the method of FIG. 4. The mixing bag can be placed on a load cell, enabling accurate determination of the mass of each concentrate flowed into the mixing bag. With a known concentration of the concentrate, the total amount of each solute flowed into the mixing bag can be controlled by the control system to result in the desired ratios of each solute in the final peritoneal dialysis fluid by flowing a predetermined mass of each concentrate into the mixing bag. In step **503,** the system can determine whether all needed concentrates have been formed. If not, the system can flow a predetermined mass of the next concentrate into the mixing bag and continue until all necessary concentrates have been added. Optionally, water can be flowed through the main fluid line of the system and into the mixing bag between addition of different concentrates. Flowing water through the fluid lines ensures that all of the concentrate has been added to the mixing bag and that no concentrate is left in the fluid lines. Depending on the concentration of solutes in the concentrates, precipitation may occur without washing water through the fluid lines between additions. Once the last concentrate to be added is added, the system can flow a predetermined mass of water into the mixing bag from the purified water bag in step **504.** To measure the amount of water flowed into the mixing bag, the system can use either or both of the load cells described. The amount of water added to the mixing bag is the amount necessary to dilute the concentrates to the desired final concentration in the peritoneal dialysis fluid. The method can end in step **505,** having generated the peritoneal dialysis fluid.

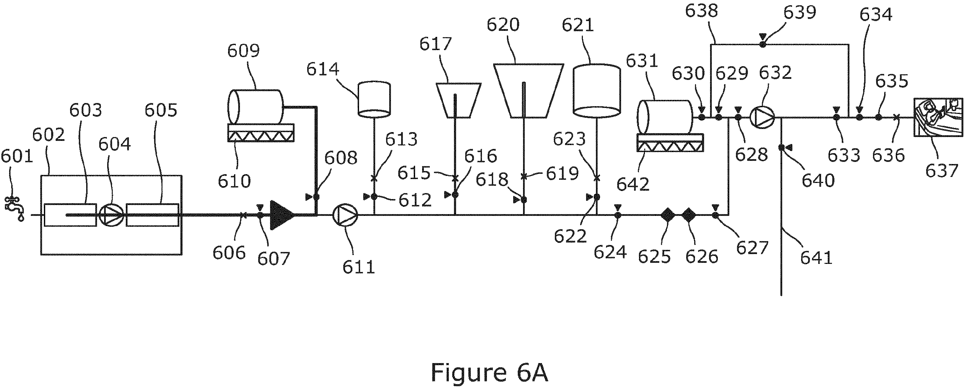

FIG.'s 6A-E illustrate the steps used to generate peritoneal dialysis fluid using a similar system to that shown in FIG. 2. FIG. 6A shows filling of a purified water bag **609.** The thick arrow shows the movement of fluid for the process. Water from a water source **601** can be flowed through a water purification module **602.** As described, the water purification module **602** can include multiple stages, including a pretreatment stage **603** and a treatment stage **605,** either of which can include multiple smaller stages. Pump **604** can provide the driving force for flowing water through water purification module **602.** To fill the purified water bag **609,** valve **607** and valve **608** can be opened. The purified water can exit water purification module **602,** flow through connector **606,** valve **607,** and valve **608,** and into purified water bag **609.** As described a load cell **610,** which can optionally include a heater, can be used to determine the mass of water added to purified water bag **609.**

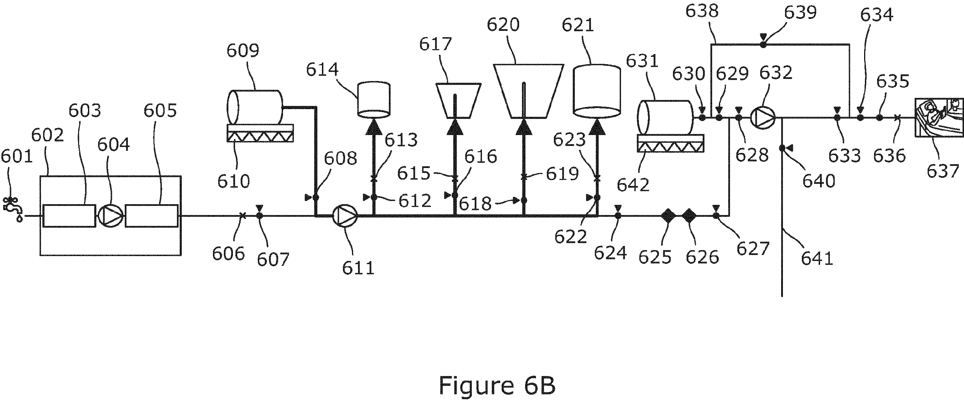

FIG. 6B shows reconstitution of dry concentrates, with the thick arrow showing the movement of fluid through the system. To reconstitute a concentrate in concentrate source **614,** valve **607** can be closed, and valve **612** opened. A predetermined mass of purified water can be flowed from purified water source **609** through valve **612** and connector **613** into concentrate source **614.** Pump **611** can provide the driving force for flowing the purified water through the system. Once a predetermined mass of fluid has been added to concentrate source **614,** valve **612** can be closed and valve **616** opened to flow purified water through connector **615** into concentrate source **617.** Valve **616** can be closed and valve **618** opened to flow purified water through connector **619** into osmotic agent source **620.** If multiple osmotic agent sources are used, valve **620** can be closed and valve **622** opened to flow purified water through connector **623** into osmotic agent source **621.** Although shown as reconstituting concentrates in each concentrate source, one of skill in the art will understand that one or more concentrate sources can be in liquid or aqueous form, and reconstitution is not necessary.

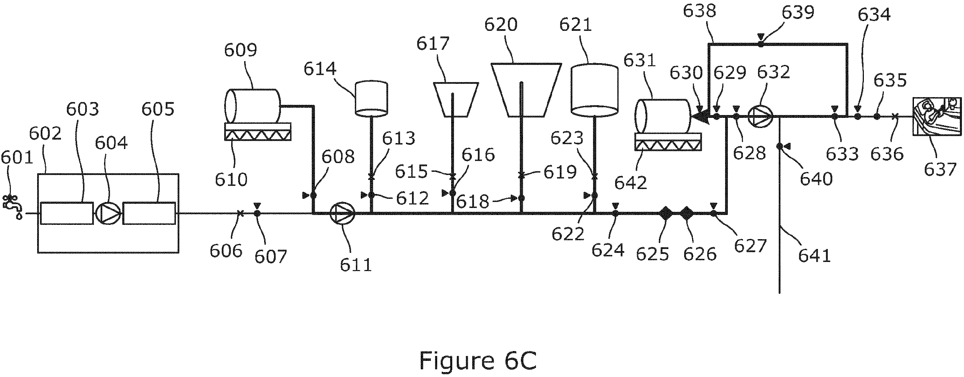

FIG. 6C shows generation of a peritoneal dialysis fluid from concentrates. Valve **612** can be opened, as well as valve **624,** valve **627,** valve **628,** valve **633,** valve **639,** and valve **630** to flow a concentrate from concentrate source **614** through the system, including fluid line **638** and into mixing bag **631.** The concentrate can pass through microbial filter **625** and microbial filter **626** to sterilize the fluids prior to addition to mixing bag **631.** Valve **612** can be closed and valve **618** opened to flow an osmotic agent concentrate from osmotic agent source **620** to mixing bag **631.** The process can be repeated to add any number of concentrates from concentrate sources to mixing bag **631.** As described, load cell and heater **642** can be used to determine the mass of each concentrate added to mixing bag **631.** Once the concentrates have been added, valve **628** can be closed and valve **629** opened to flow purified water from purified water bag **209** to the mixing bag **631** to dilute the peritoneal dialysis fluid to the appropriate concentration.

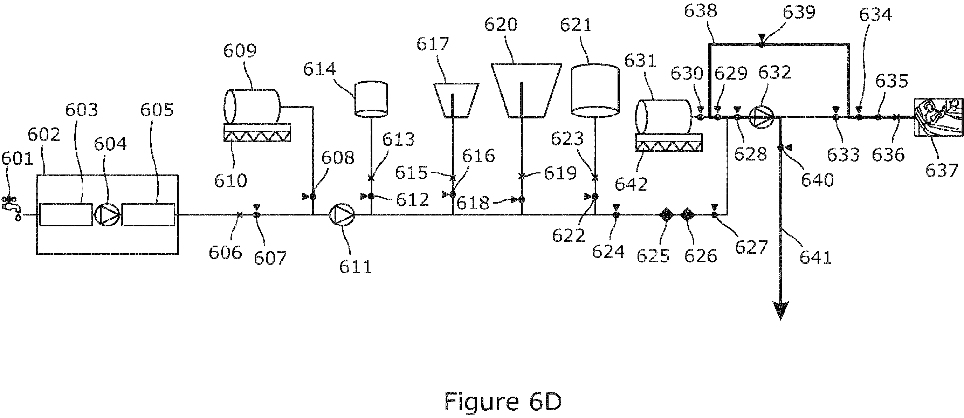

FIG. 6D shows draining of spent peritoneal dialysis fluid from a patient **637.** Valve **627** and valve **633** can be closed, and valve **634,** valve **639,** valve **629,** and valve **640** opened to flow spent peritoneal dialysis fluid from patient **637,** through connector **635,** connector **636** and fluid line **638** to drain line **641.** Pump **632** can provide the driving force for flowing fluid out of and into the patient **637.**

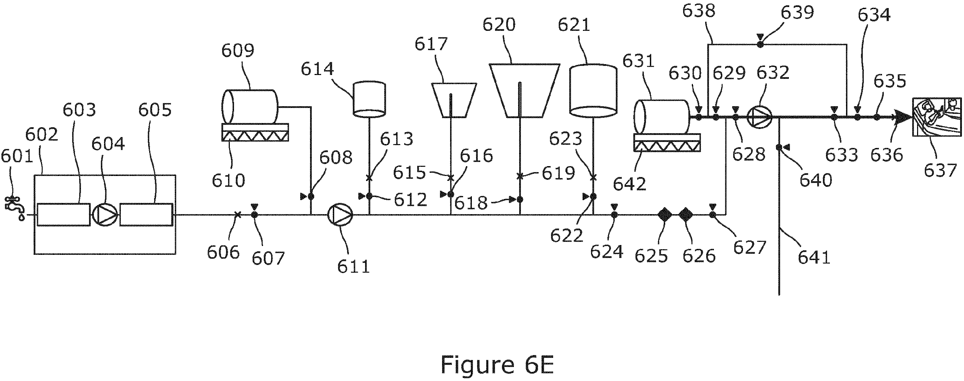

FIG. 6E shows filling of the patient **637.** Valve **639** and valve **640** can be closed, and valve **630,** valve **629,** valve **628,** valve **633,** and valve **634** can be opened. Pump **632** can flow the peritoneal dialysis fluid from mixing bag **631** into the peritoneal cavity of the patient **637** through connector **636.** One of skill in the art will understand that different valve and pump arrangements can be used besides that shown in FIG.'s 6A-E, and that FIG.'s 6A-E are provided to show one non-limiting embodiment.

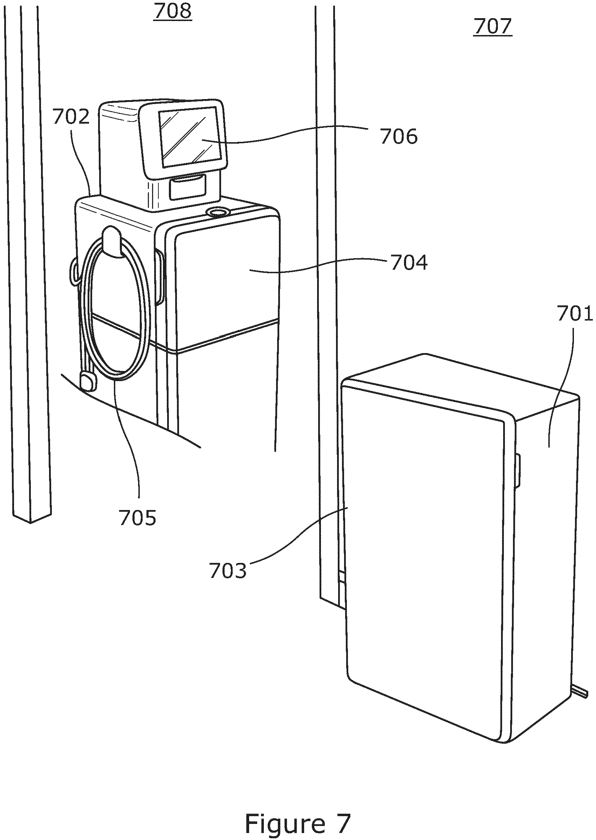

FIG. 7 is a non-limiting example of a peritoneal dialysis fluid generation system set up for use. A water purification module **701** can be connected to a water source (not shown), such as a tap water source of a house. Water can be flowed into the water purification module **701** and purified to remove any contaminants in the water source. As described, pressure generated by the water source can provide the driving force for flowing water into the water purification module **701.** Alternatively, a pump can be used to flow the water through the system. Door **703** in water purification module **701** can be opened to obtain access to the components of the water purification module **701** for maintenance or replacement.

The system can also include a peritoneal dialysis fluid generation module and cycler **702.** As described, the system can include a purified water bag (not shown) for storing purified water prior to use. The purified water bag can be positioned as part of either the water purification module **701** or the peritoneal dialysis fluid generation module and cycler **702.** A fluid line **705** can be connected to the water purification module **701** to allow purified water to be flowed from water purification module **701** to peritoneal dialysis fluid generation module and cycler **702.** Alternatively, the user may be able to remove the purified water bag from water purification module **701** and place the purified water bag close to the peritoneal dialysis fluid generation module and cycler **702** for connection.

As described, the components of the peritoneal dialysis fluid generation module and cycler **702** can be all or partly disposable. Door **704** on peritoneal dialysis fluid generation module and cycler **702** can be opened to connect a consumable cassette and/or the concentrate sources. A mixing bag (not shown) can be included in peritoneal dialysis fluid generation module and cycler **702** to receive the components of the peritoneal dialysis fluid. A heater (not shown) can heat the peritoneal dialysis fluid prior to infusion into the patient through a disposable transfer set (not shown).

Graphical user interface **706** can be included on either or both of the water purification module **701** and peritoneal dialysis fluid generation module and cycler **702.** Through the graphical user interface **706,** the user can control the water purification, peritoneal dialysis fluid generation, and treatment. The user can also receive messages and/or alerts. The peritoneal dialysis fluid generation module and cycler **702** can communicate with water purification module **701** through any means known in the art, including Bluetooth, Wi-Fi, or wired communication to coordinate the water purification, peritoneal dialysis fluid generation, and treatment. In certain embodiments fluid line **705** can be long enough for water purification module **701** and peritoneal dialysis fluid generation module and cycler **702** to be placed in separate rooms. As illustrated in FIG. 7, water purification module **701** can be placed in a first room **707,** such as a bathroom, while peritoneal dialysis fluid generation module and cycler **702** is placed in a second room **708,** such as a bedroom. However, one of skill in the art will understand that a shorter fluid line can be used, and the modules placed closer together.

One skilled in the art will understand that various combinations and/or modifications and variations can be made in the described systems and methods depending upon the specific needs for operation. Various aspects disclosed herein may be combined in different combinations than the combinations specifically presented in the description and accompanying drawings. Moreover, features illustrated or described as being part of an aspect of the disclosure may be used in the aspect of the disclosure, either alone or in combination, or follow a preferred arrangement of one or more of the described elements. Depending on the example, certain acts or events of any of the processes or methods described herein may be performed in a different sequence, may be added, merged, or left out altogether (e.g., certain described acts or events may not be necessary to carry out the techniques). In addition, while certain aspects of this disclosure are described as performed by a single module or unit for purposes of clarity, the techniques of this disclosure may be performed by a combination of units or modules associated with, for example, a medical device.

## Claims

1. A system, comprising:
a water purification module fluidly connectable to a water source;
a disposable cassette;
the disposable cassette comprising a first connector fluidly connectable to the water purification module; the disposable cassette further comprising:
a) a first fluid line fluidly connected to the first connector;
b) at least one concentrate line fluidly connectable to a first concentrate source and fluidly connected to the first fluid line;
c) at least one osmotic agent line fluidly connectable to a first osmotic agent source and fluidly connected to the first fluid line; and
d) at least a second connector fluidly connectable to a patient.

2. The system of claim 1, the disposable cassette further comprising at least one microbial filter.

3. The system of claim 1, the disposable cassette further comprising at least one pump.

4. The system of claim 1, the disposable cassette further comprising a mixing bag fluidly connected to the first fluid line.

5. The system of claim 1, the disposable cassette further comprising a purified water bag fluidly connected to the first fluid line upstream of the at least one concentrate line and at least one osmotic agent line.

6. The system of claim 5, further comprising a heater for heating the purified water bag.

7. The system of claim 1, wherein the at least one osmotic agent line comprises a first osmotic agent line fluidly connectable to a first osmotic agent source and a second osmotic agent line fluidly connectable to a second osmotic agent source.

8. The system of claim 1, wherein the at least one concentrate line comprises a first concentrate line fluidly connectable to a first concentrate source and a second concentrate line fluidly connectable to a second concentrate source.

9. The system of claim 1, wherein the water source is a drinkable water source.

10. The system of claim 1, further comprising a receiving compartment for receiving the disposable cassette.

11. A method of using the system of claim 1, comprising the steps of:
flowing water from the water source through the water purification module and into the first fluid line;
flowing a concentrate from the at least one concentrate source into the first fluid line;
flowing an osmotic agent concentrate from the at least one osmotic agent source into the first fluid line; and
flowing fluid from the first fluid line into a mixing bag.

12. The method of claim 11, further comprising the step of determining a mass of fluid in the mixing bag; and wherein the step of flowing the concentrate into the first fluid line comprises flowing the concentrate until a mass of fluid in the mixing bag increases by a preset amount; and wherein the step of flowing the osmotic agent concentrate into the first fluid line comprises flowing the osmotic agent concentrate until a mass of fluid in the mixing bag increases by a preset amount.

13. The method of claim 11, further comprising the step of heating the mixing bag after flowing the fluid from the first fluid line into the mixing bag.

14. The method of claim 11, further comprising the step of flowing water into the at least one concentrate source to generate the concentrate and flowing water into the at least one osmotic agent source to generate the osmotic agent concentrate.

15. The method of claim 14, further comprising the step of flowing water from the water purification module to a purified water bag prior to flowing the water into the at least one concentrate source and the at least one osmotic agent source.

16. The method of claim 15, further comprising the step of determining a mass of water in the purified water bag; wherein the step of flowing water into the at least one concentrate source comprises flowing a predetermined mass of water into the at least one concentrate source; and wherein the step of flowing water into the at least one osmotic agent source comprises flowing a predetermined mass of water into the at least one osmotic agent source.

17. A method of using the system of claim 1, comprising the steps of flowing water from the water source through the water purification module;
flowing the water from the water purification module into a purified water bag fluidly connected to the first fluid line;
flowing water from the purified water bag into the at least one concentrate source to generate a concentrate; and
flowing water from the purified water bag into the at least one concentrate source to generate an osmotic agent concentrate.

18. The method of claim 17, wherein the at least one concentrate source initially contains solid material, and wherein the at least one osmotic agent source initially contains solid material.

19. The method of claim 17, further comprising the step of determining a mass of water in the purified water bag prior to flowing the water into the at least one concentrate source and the at least one osmotic agent source.

20. The method of claim 19, wherein the step of flowing water from the purified water bag to the at least one concentrate source comprises flowing the water until the mass of water in the purified water bag decreases by a preset amount.

21. The method of claim 19, wherein the step of flowing water from the purified water bag to the at least one osmotic agent source comprises flowing the water until the mass of water in the purified water bag decreases by a preset amount.
